# EUROPEAN PATENT APPLICATION

(11) **EP 3 616 633 A1**
(43) Date of publication of application: **04.03.2020**
(21) Application number: 19193447.0
(22) Date of filing: 23.08.2019
(51) Int. Cl.: A61B 17/34, A61B 17/00, A61B 17/04

(54) **SURGICAL ACCESS SLEEVE AND SURGICAL ACCESS DEVICE COMPRISING THE SAME**

(30) Priority: 29.08.2018 TW 107130108
(71) Applicant: Hwealthy Co., LTD., Taichung City (TW)
(72) Inventor: KU, KUN-TAI, Taichung City (TW); CHANG, SHIH-CHIEH, Taichung City (TW)
(74) Representative: 2K Patentanwälte Blasberg Kewitz & Reichel

(57) **Abstract**

A surgical access sleeve comprises a trocar sleeve (20). The trocar sleeve (20) includes: a trocar housing (22) including a housing base (22a) and a housing cover (22b) disposed on the housing base (22a), the housing base (22a) including an annular wall (221), a lower chamber (222) disposed inside the annular wall (221) and a first guide channel (2211) obliquely penetrating through the annular wall (221) but being not communicated with the lower chamber (222); and a trocar cannula (21) including a first open end (21a) connected with the trocar housing (22), a second open end (21b) located opposite to the first open end (21a), and a tube wall (23) disposed between the first open end (21a) and the second open end (21b), the tube wall (23) including a first upper guide through hole (23a) and a first lower guide through hole (23b), wherein the first guide channel (2211), the first upper guide through hole (23a), and the first lower guide through hole (23b) are coaxially disposed along a virtual axis (25).

## Description

### FIELD OF THE INVENTION

The present invention relates to a surgical access sleeve and a surgical access device comprising the same, in particular to a surgical access sleeve and a surgical access device comprising the same for minimally invasive suture surgeries.

### BACKGROUND OF THE INVENTION

With the increasing prevalence of minimally invasive surgeries, instruments applied to the minimally invasive surgeries have evolved accordingly, for example, a surgical access sleeve is a commonly used instrument for minimally invasive surgeries. An operator needs to cut an incision on a patient through a minimally invasive surgical instrument and perform a surgery on the incision. According to different lesions, the size and depth of the incision are different. During a posterior procedure of the surgery, suturing of the incision is inevitably required. As the incision cut in the minimally invasive surgery is different from an incision cut in a conventional surgery in size and depth, the incision cannot be sutured by a common suturing method.

However, stability is important during suturing. In the minimally invasive surgery, error-tolerant rate associated of suturing the incision is reduced since the incision is relatively small. A slight carelessness or accident may expose the patient to a high risk, or even irreversible injury.

In the related prior art of the surgical access sleeve, an invasive surgical device as described in the European Patent Publication No. EP 3225202 A1, is a type of the surgical access sleeve, wherein part of assemblies and an inner chamber are of a communicated structure. However, the communicated structure causes the problem of insufficient airtightness, and the processing difficulty is increased by its complicated structural design. Moreover, a cannula as described in the U.S. Patent No. 9,033,872 B2 is provided for using in surgery and its structure is simple relative to the related prior art of the surgical access sleeve. Nevertheless, it is less convenient that an operator needs to remove a housing while using. Accordingly, the surgical access sleeve remains to be improved.

### SUMMARY OF THE INVENTION

A main object of the present invention is to improve the operational problem of a surgical access sleeve in the prior art in use.

In order to achieve the object, the present invention provides a surgical access sleeve which comprises a trocar sleeve, and the trocar sleeve comprises a trocar housing including a housing base and a housing cover disposed on the housing base. The housing base includes an annular wall, a lower chamber disposed inside the annular wall and a first guide channel obliquely penetrating through the annular wall but being not communicated with the lower chamber, and the housing cover includes a hole disposed coaxially with the lower chamber. Moreover, a trocar cannula including a first open end connected with the trocar housing, a second open end located opposite to the first open end, and a tube wall disposed between the first open end and the second open end. In addition, an inner chamber is defined by the tube wall, and the tube wall includes a first upper guide through hole and a first lower guide through hole, wherein the first guide channel, the first upper guide through hole, and the first lower guide through hole are coaxially disposed along a virtual axis, and an angle of 10 degrees to 35 degrees is formed between the virtual axis and the tube wall. An airtight film is disposed on the tube wall of the trocar cannula and covers the first upper guide through hole.

The present invention further provides a surgical access device comprising a surgical access assembly and a surgical access sleeve, wherein the surgical access assembly comprises an operating portion and a needle body assembled with the operating portion. The surgical access sleeve comprises a trocar sleeve, and the trocar sleeve comprises a trocar housing including a housing base and a housing cover disposed on the housing base. The housing base includes an annular wall, a lower chamber disposed inside the annular wall and a first guide channel obliquely penetrating through the annular wall but being not communicated with the lower chamber, and the housing cover includes a hole disposed coaxially with the lower chamber. Moreover, a trocar cannula includes a first open end connected with the trocar housing, a second open end located opposite to the first open end, and a tube wall disposed between the first open end and the second open end. In addition, an inner chamber is defined by the tube wall, and the tube wall includes a first upper guide through hole and a first lower guide through hole. Further, after the needle body is assembled on the surgical access sleeve by penetrating through the first upper guide through hole and the first lower guide through hole, the first guide channel, the first upper guide through hole, and the first lower guide through hole are arranged in a row on the needle body, and an angle of 10 degrees to 35 degrees is formed between the needle body and the tube wall. An airtight film is disposed on the tube wall of the trocar cannula and covering the first upper guide through hole.

Accordingly, based on the design of the first guide channel, the surgical access sleeve or the surgical access device of the present invention improves stability of an operator in suturing an incision, and avoids shaking of hands during operation that affects the suturing process. Furthermore, extra wounds to a patient caused during suturing are avoided due to the increase of the stability, so that the recovery rate of the patient is improved, and the infection probability is reduced.

### BRIEF DESCRIPTION OF THE DRAWINGS

Fig. 1 is a schematic perspective view of appearance of an embodiment of a surgical access device of the present invention.
Fig. 2A is a schematic partial cross-sectional side view of an embodiment of the surgical access device of the present invention.
Fig. 2B is a enlarged partial view of a first guide channel of Fig. 2A.
Fig. 3 is a schematic partial cross-sectional side view of another embodiment of the surgical access device of the present invention.
Fig. 4 is a schematic perspective view of appearance of another embodiment of the surgical access device of the present invention.
Fig. 5 is a schematic perspective view of appearance of the surgical access device during puncturing in an embodiment of the surgical access device of the present invention.
Fig. 6A and Fig. 6B are schematic diagrams illustrating a first clamping portion and a second clamping portion being clamped together in an embodiment of the surgical access device of the present invention.
Fig. 7A and Fig. 7B are schematic diagrams illustrating practical application in an embodiment of the surgical access device of the present invention.

### DETAILED DESCRIPTION OF THE PREFERRED EMBODIMENTS

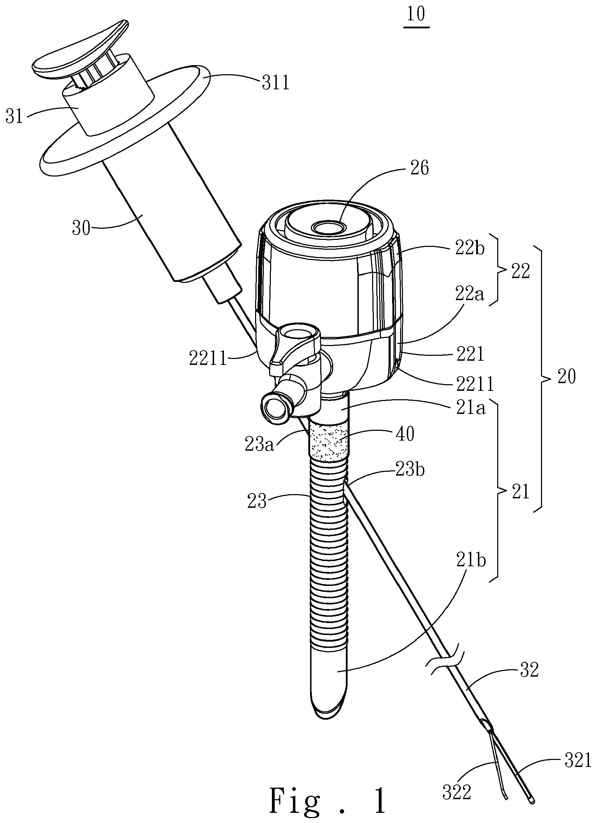

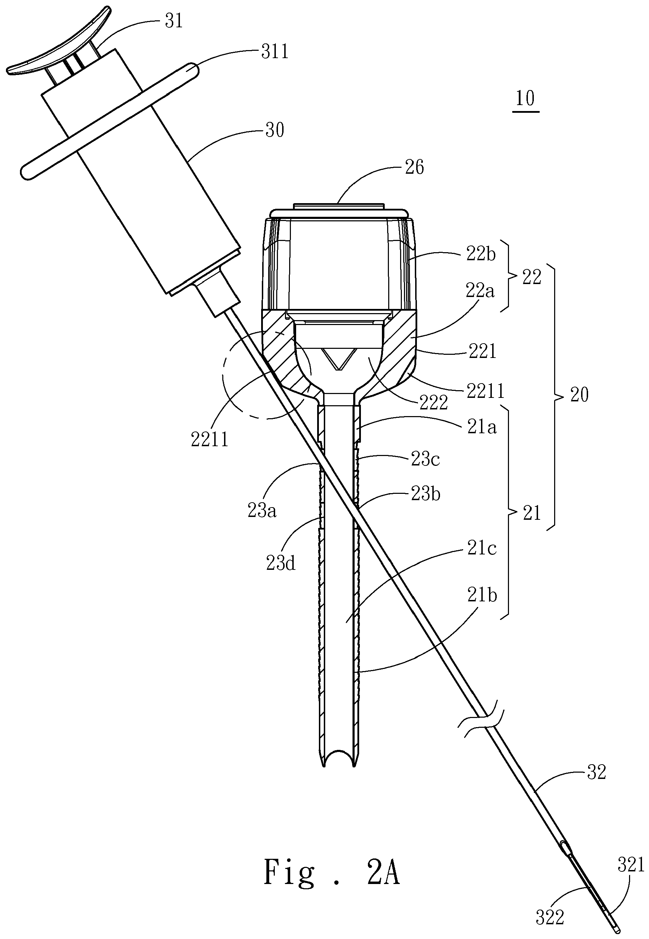

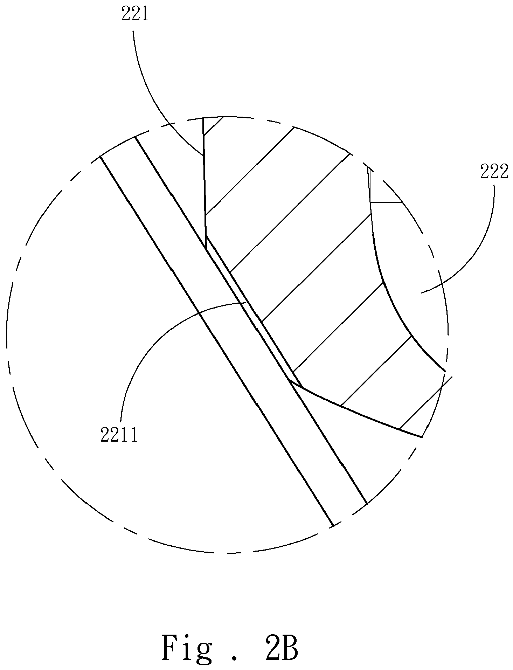

The detailed description and technical contents of the present invention are now described below in conjunction with the accompanying drawings:
Referring to Fig. 1, Fig. 2A and Fig. 2B, the present invention relates to a surgical access device 10 generally comprising a surgical access assembly 30 and a surgical access sleeve including a trocar sleeve 20.

Firstly, the surgical access sleeve mainly includes the trocar sleeve 20, and the trocar sleeve 20 includes a trocar cannula 21 and a trocar housing 22 assembled with the trocar cannula 21. The trocar housing 22 includes a housing base 22a and a housing cover 22b disposed on the housing base 22a. The housing base 22a includes an annular wall 221, a lower chamber 222 disposed inside the annular wall 221 and a first guide channel 2211 obliquely penetrating through the annular wall 221 but being not communicated with the lower chamber 222, and the housing cover 22b includes a hole 26 disposed coaxially with the lower chamber 222. In the embodiment, as shown in Fig. 1 and Fig. 2A, a pair of first guide channels 2211 is symmetrically formed in the housing base 22a. In other embodiment, the annular wall 221 may include an oblique guide wall, and the first guide channel 2211 is disposed in the oblique guide wall. In addition, the annular wall 221 may also include an oblique guide groove, and the first guide channel 2211 is disposed in the oblique guide groove. However, the embodiments described above are merely exemplary, without limiting the possibility of other aspects.

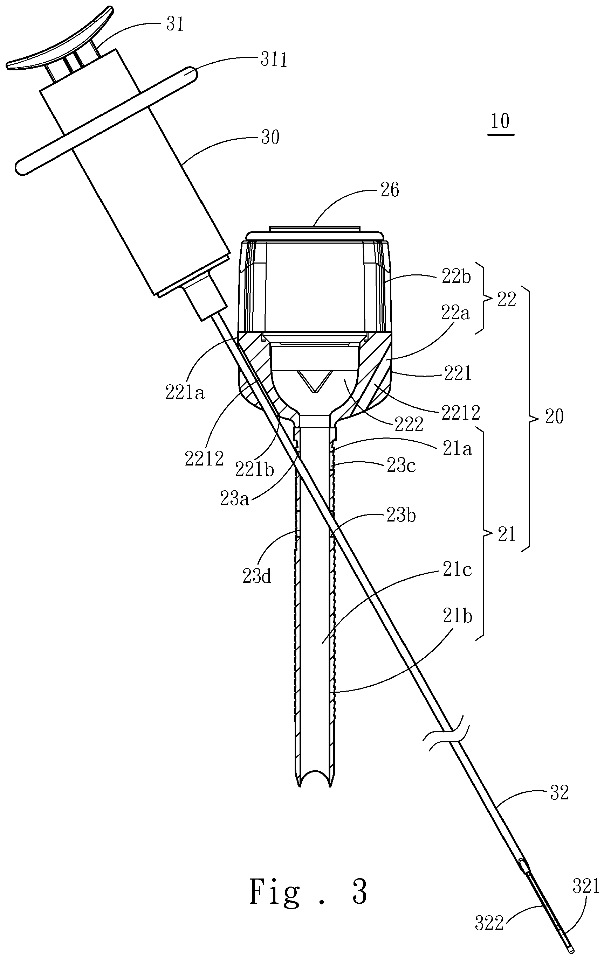

In another embodiment, further refer to Fig. 3, the annular wall 221 includes a side wall 221a, a bottom wall 221b joined with the side wall 221a, and a guide hole 2212 penetrating from the side wall 221a to the bottom wall 221b. Moreover, the guide hole 2212 does not penetrate through the lower chamber 222.

Referring to Fig. 1 and Fig. 2A, the trocar cannula 21 includes a first open end 21a connected with the trocar housing 22, a second open end 21b located opposite to the first open end 21a, and a tube wall 23 disposed between the first open end 21a and the second open end 21b and defining an inner chamber 21c. The tube wall 23 includes a first upper guide through hole 23a and a first lower guide through hole 23b, which are disposed obliquely corresponding to each other and penetrates through the inner chamber 21c. The trocar housing 22 includes a housing base 22a connected with the first open end 21a and a housing cover 22b located opposite to the first open end 21a.

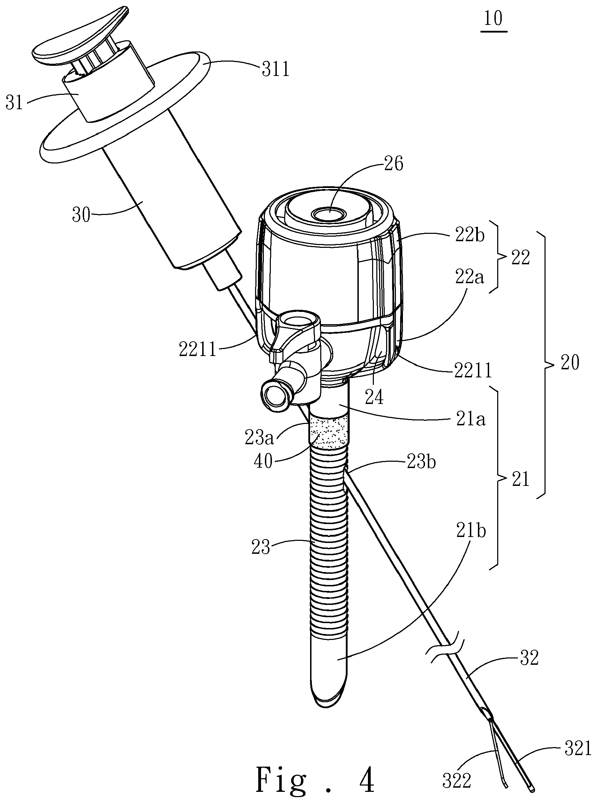

In addition, as shown in Fig. 4, the housing base 22a further includes at least one pair of external lugs 24 respectively disposed on two opposite side edges of the housing base 22a , and the first guide channel 2211 is formed on each external lug 24. In the embodiment, the external lugs 24 have structures in L-like shapes, but are not limited thereto, and in other embodiments, the external lugs 24 may have more rounded structures, such as semicircular structures, or multiangular structures, such as rectangles. Diversified designs may be made depending on operating habits of those skilled in the art.

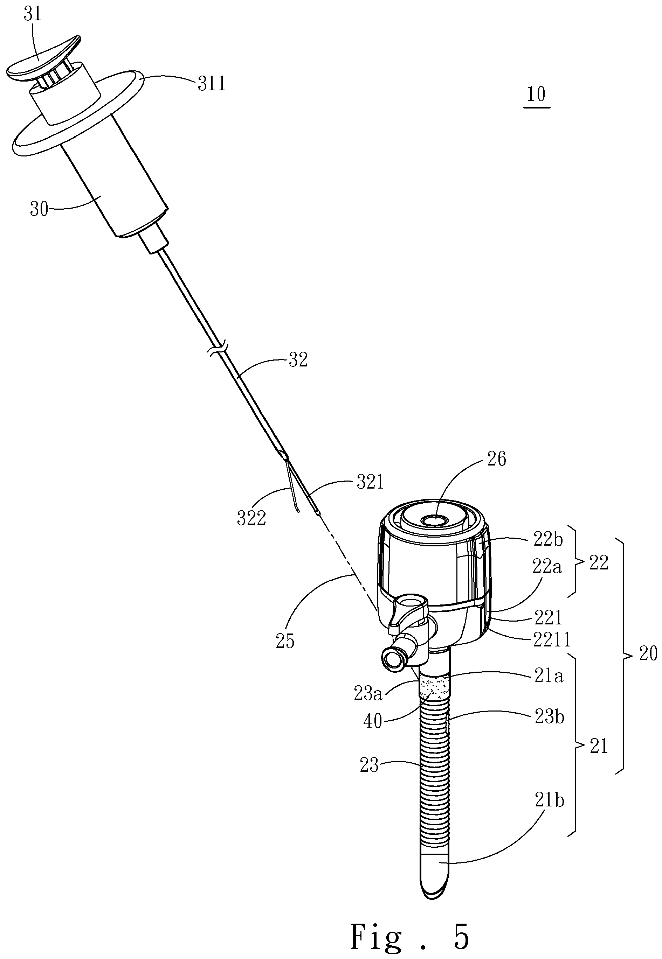

Referring to Fig. 5, the first guide channel 2211, the first upper guide through hole 23a, and the first lower guide through hole 23b are arranged in a row on a virtual axis 25, that is, the first guide channel 2211, the first upper guide through hole 23a, and the first lower guide through hole 23b are coaxial on the virtual axis 25, and an angle of 10 degrees to 35 degrees is formed between the virtual axis 25 and the tube wall 23.

In addition to the first upper guide through hole 23a and the first lower guide through hole 23b, the number of "guide through hole" may be plural. For example, a second upper guide through hole 23c and a second lower guide through hole 23d correspondingly disposed to the second upper guide through hole 23c may be additionally disposed. Similar to the first upper guide through hole 23a, the first lower guide through hole 23b and the first guide channel 2211 are coaxial on the virtual axis 25 as described above, the second upper guide through hole 23c, the second lower guide through hole 23d and the first guide channel 2211 are also coaxial on another virtual axis. For convenience of operation, in addition to the shape, number, and position, the diameter and size of the guide through holes described above may also be different designs, the embodiments described above which could be realized by those of ordinary skill in the art will not be described in further detail herein.

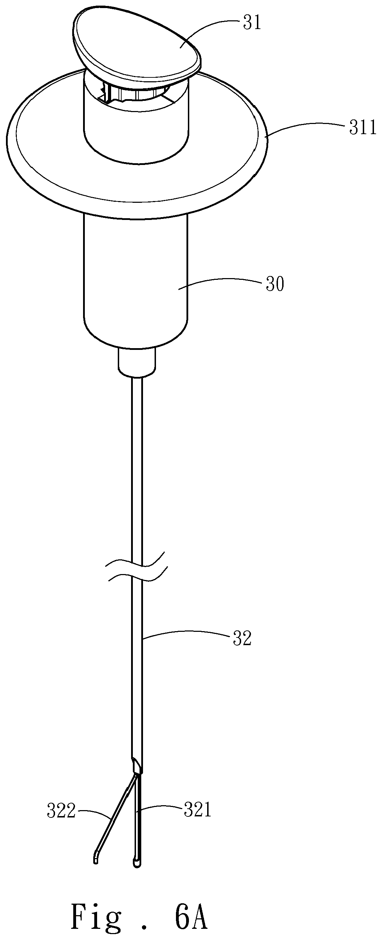

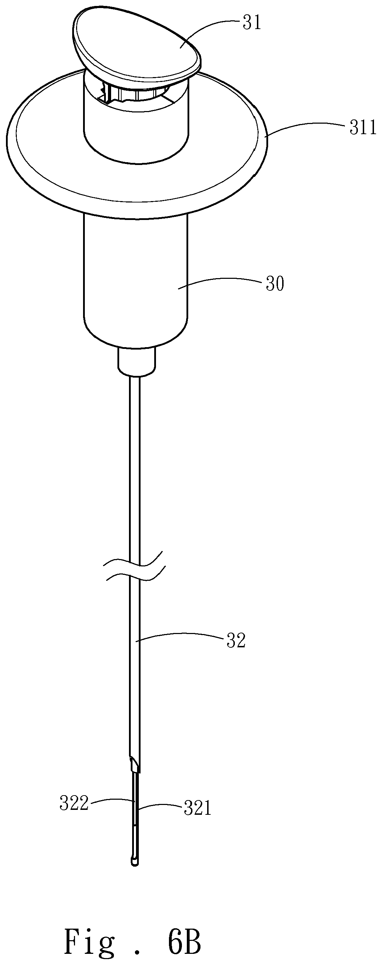

It is understandable to those of ordinary skill in the art that the surgical access device 10 of the present invention not only includes the surgical access sleeve as mentioned above, but also includes a surgical access assembly 30. Please refer to Fig. 5, the surgical access assembly 30 includes an operating portion 31 and a needle body 32 assembled with the operating portion 31. In a preferred embodiment, a side edge of the operating portion 31 comprises a blocking portion 311 extending outwardly in an axial direction of the operating portion 31, and the blocking portion 311 is provided for a person skilled in the art to hold the surgical access assembly 30 more stably for facilitating operation and use. The blocking portion 311 may be an annular sheet structure, a rectangular-like sheet structure and the like, which is not particularly limited. Further, as shown in Fig. 6A and Fig. 6B, the needle body 32 includes a first clamping portion 321 and a second clamping portion 322 which are controlled to be opened and closed by the operating portion 31.

The needle body 32 penetrates through the first upper guide through hole 23a and the first lower guide through hole 23b along the virtual axis 25 to assembled on the trocar sleeve 20. After the needle body 32 is assembled on the trocar sleeve 20 by penetrating through the first upper guide through hole 23a and the first lower guide through hole 23b along the virtual axis, the first guide channel 2211, the first upper guide through hole 23a and the first lower guide through hole 23b are arranged in a row on the needle body 32, so that an angle of 10 degrees to 35 degrees is formed between the needle body 32 and the tube wall 23. In the present invention, the shapes of the first upper guide through hole 23a and the first lower guide through hole 23b are not limited and may be designed in consideration of the operational mobility of the needle body 32. In an embodiment of the present invention, the first upper guide through hole 23a and the first lower guide through hole 23b are elliptical in shape, but are not limited thereto, and the needle body 32 may have a greater degree of freedom in operation by matching with an included angle between the virtual axis 25 and the trocar cannula 21.

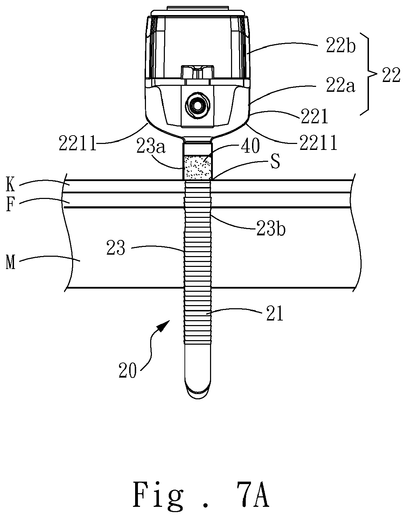

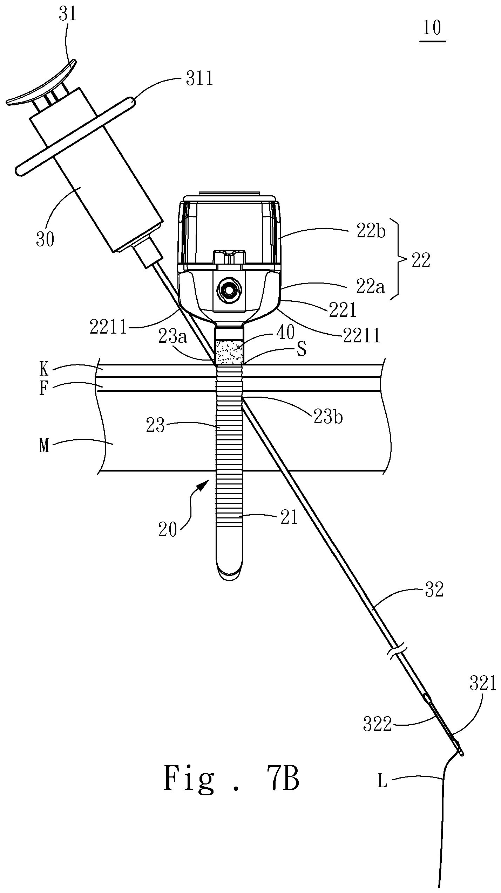

Referring to Fig. 7A and Fig. 7B together, an operation method for the surgical access device 10 of the present invention applied to minimally invasive surgical suturing is described as follow.

It is understandable to those of ordinary skill in the art that a suture L is used for suturing the incision during the surgery and is wound around the needle body 32.

Firstly, the trocar cannula 21 of the trocar sleeve 20 is inserted directly into a notch S formed in a minimally invasive surgery until the first lower guide through hole 23b reaches the bottom of a muscle layer M or other suturing sites such as a skin layer K or a fat layer F. In the present invention, the bottom of the muscle layer M is used as an example, but not limited to. The trocar sleeve 20 may be rotated so that the first lower guide through hole 23b is positioned at a site where a suturing surgery is optimally performed, depending on the type of the notch S or the suturing method. As in the present invention, the tube wall 23 is a screw thread structure or a rough structure, so that the trocar cannula 21 may be prevented from falling off in use as well as being pushed inwards during the surgery.

The surgical access assembly 30 clamps the suture L with the first clamping portion 321 and the second clamping portion 322. It is understandable for those skilled in the art that removal of the suture during the surgery may result in failure of suturing or a residual suture falling into a patient's body, creating a dangerous situation. After the suture L is clamped by the first clamping portion 321 and the second clamping portion 322, the needle body 32 penetrates through the first upper guide through hole 23a and the first lower guide through hole 23b, and the needle body 32 is guided to a suturing site by the first lower guide through hole 23b for suturing. In addition, as the guide through hole positioned above, for instance, the first upper guide through hole 23a is exposed outside a human body during the surgery. Thus, it is necessary to seal the first upper guide through hole 23a with an airtight film 40 disposed on the tube wall 23. The airtight film 40 is made of a puncturable material. During suturing, the needle body 32 punctures the airtight film 40 and then penetrates through the first upper guide through hole 23a and the first lower guide through hole 23b. As the second upper guide through hole 23c is further included as described above, the second upper guide through hole 23c positioned above may also be sealed with the airtight film 40. In other embodiment, the airtight film 40 may further cover the first lower guide through hole 23b and the second lower guide through hole 23d together.

It is understandable to those of ordinary skill in the art that the stability of the surgical access assembly 30 is an important factor during the suturing surgery. Unlike common surgeries, a slight carelessness or accident in the minimally invasive surgery may cause additional injury to the patient since the incision is relatively small. Thus, the first guide channel 2211 designed in the present invention is provided for the surgical access assembly 30 abuts the first guide channel 2211, and an outer edge of the needle body 32 may abut against the first guide channel 2211 and penetrate through the first upper guide through hole 23a and the first lower guide through hole 23b, so that the operation stability in a suturing process is greatly improved.

In summary, compared with the prior art, on the basis of the design of the first guide channel, and the corresponding arrangement of the first guide channel, the first upper guide through hole and the first lower guide through hole, the surgical access device of the present invention will improve the stability of an operator in suturing the incision, and avoid hand shaking during operation, thereby preventing from being affected in the suturing surgeries. Furthermore, additional wounds to the patient are prevented during suturing surgeries due to the improvement of the stability, so that the recovery rate of the patient is improved, and the infection probability is reduced.

## Claims

1. A surgical access sleeve comprising:
a trocar sleeve (20) including:
a trocar housing (22) including a housing base (22a) and a housing cover (22b) disposed on the housing base (22a), the housing base (22a) including an annular wall (221), a lower chamber (222) disposed inside the annular wall (221) and a first guide channel (2211) obliquely penetrating through the annular wall (221) but being not communicated with the lower chamber (222), and the housing cover (22b) including a hole (26) disposed coaxially with the lower chamber (222);
a trocar cannula (21) including a first open end (21a) connected with the trocar housing (22), a second open end (21b) located opposite to the first open end (21a), and a tube wall (23) disposed between the first open end (21a) and the second open end (21b), an inner chamber (21c) defined by the tube wall (23), and the tube wall (23) including a first upper guide through hole (23a) and a first lower guide through hole (23b), wherein the first guide channel (2211), the first upper guide through hole (23a), and the first lower guide through hole (23b) are coaxially disposed along a virtual axis (25), and an angle of 10 degrees to 35 degrees is formed between the virtual axis (25) and the tube wall (23); and
an airtight film (40) disposed on the tube wall (23) of the trocar cannula (21) and covering the first upper guide through hole (23a).

2. A surgical access device (10) comprising a surgical access assembly (30) and a surgical access sleeve, the surgical access assembly (30) including an operating portion (31) and a needle body (32) assembled with the operating portion (31), and the surgical access sleeve including:
a trocar sleeve (20) including:
a trocar housing (22) including a housing base (22a) and a housing cover (22b) disposed on the housing base (22a), the housing base (22a) including an annular wall (221), a lower chamber (222) disposed inside the annular wall (221) and a first guide channel (2211) obliquely penetrating through the annular wall (221) but being not communicated with the lower chamber (222), and the housing cover (22b) including a hole (26) disposed coaxially with the lower chamber (222);
a trocar cannula (21) including a first open end (21a) connected with the trocar housing (22), a second open end (21b) located opposite to the first open end (21a), and a tube wall (23) disposed between the first open end (21a) and the second open end (21b), an inner chamber (21c) defined by the tube wall (23), and the tube wall (23) including a first upper guide through hole (23a) and a first lower guide through hole (23b), wherein after the needle body (32) is assembled on the surgical access sleeve by penetrating through the first upper guide through hole (23a) and the first lower guide through hole (23b), the first guide channel (2211), the first upper guide through hole (23a), and the first lower guide through hole (23b) are arranged in a row on the needle body (32), and an angle of 10 degrees to 35 degrees is formed between the needle body (32) and the tube wall (23); and
an airtight film (40) disposed on the tube wall (23) of the trocar cannula (21) and covering the first upper guide through hole (23a).

3. The surgical access sleeve or the surgical access device (10) according to claim 1 or 2, wherein the annular wall (221) includes an oblique guide wall, the first guide channel (2211) is disposed in the oblique guide wall.

4. The surgical access sleeve or the surgical access device (10) according to claim 1 or 2, wherein the annular wall (221) includes an oblique guide groove, the first guide channel (2211) is disposed in the oblique guide groove.

5. The surgical access sleeve or the surgical access device (10) according to claim 1 or 2, wherein the annular wall (221) includes a side wall (221a), a bottom wall (221b) joined with the side wall (221a) and a guide hole (2212) penetrating from the side wall (221a) to the bottom wall (221b).

6. The surgical access sleeve or the surgical access device (10) according to claim 5, wherein the guide hole (2212) does not penetrate through the lower chamber (222).

7. The surgical access sleeve or the surgical access device (10) according to claim 1 or 2, wherein the housing base (22a) further includes at least one pair of external lugs (24) respectively disposed on two opposite side edges of the housing base (22a), and the first guide channel (2211) is formed on each external lug (24).
